(19) Europäisches Patentamt
European Patent Office
Office européen des brevets

(11) **EP 2 671 613 A2**

(12) **EUROPEAN PATENT APPLICATION**
published in accordance with Art. 153(4) EPC

(43) Date of publication:
**11.12.2013 Bulletin 2013/50**

(51) Int Cl.:
*A61N 2/00* (2006.01)       *A61N 2/02* (2006.01)
*A61N 1/32* (2006.01)       *A61B 18/04* (2006.01)
*A61L 31/02* (2006.01)

(21) Application number: **12742595.7**

(22) Date of filing: **31.01.2012**

(86) International application number:
**PCT/KR2012/000742**

(87) International publication number:
**WO 2012/105796 (09.08.2012 Gazette 2012/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.02.2011 KR 20110010114**

(71) Applicant: **Korea University Research And
Business Foundation
Seoul 136-713 (KR)**

(72) Inventors:
• **CHOI, Sung Hoi**
  **Moscow, Idaho 83843 (US)**
• **PARK, Jung Rae**
  **Andover, Massachusetts 01810 (US)**
• **KIM, Young Keun**
  **Seoul 135-906 (KR)**

(74) Representative: **Eisenführ, Speiser & Partner
Postfach 10 60 78
28060 Bremen (DE)**

(54) **ROTATING NANOWIRE AND METHOD FOR INDUCING CELL NECROSIS USING SAME**

(57) The present invention provides a cell eradication method and a cell eradication principle for necrotizing a cell by agitating a cell using a physical turning force from the impression of an AC magnetic field, after preparing a magnetic nanowire having a dipole and introducing the magnetic nanowire into a cell. Therefore, the composition for inducing cell eradication of the present invention, when applied to a cell that is requested to be removed such as a cancer cell, can eradicate the cell by applying a physical impact through the rotation of the nanowire introduced inside the cell. Additionally, the heat generated from induced current from the magnetic field impression can add an effect of thermotherapy, and also, attaching a drug to the surface of the nanowire enhances the treatment effects.

【FIG. 4】

EP 2 671 613 A2

**Description**

[Technical Field]

The present invention relates to a technology for physically eradicatingcells which need to be removed, such as cancer cells, using a rotation force of a nanowire, namely to a nanowire which can be used for inducing cell eradication.

[Background Art]

Efforts by both academics and industries to create a new technology domain called nanomedical technology by integrating advantages of nanotechnology and medical science have been made. The technologies related to a nanostructure have a wide range of applications, such as electronic devices, magnetic devices, catalysts, medical diagnosis treatments, and so on. Especially, in case of a nanowire produced using a nanotemplate, its size, shape, and crystallinity can be easily controlled, and thus many attentions have been paid to it. Indeed, the application of a nanowire has a quite wide scope including nano-electromechanical system, advanced high density magnetic memory, fuel cell, nano-biosensor, cell separation, and so on. A nanomagnet denotes a 3-dimensional structured body with magnetic properties having a globe, linear, or tube form wherein a minimum unit of one dimension (thickness, diameter, or length) is no more than several hundred nanometers. Such a nanomagnet is used as an advanced material, such as a diagnosis reagent for nucleic acid and protein, a contrast medium for magnetic resonance imaging (MRI), and a heating agent for malignant cells. Furthermore, the nanomagnet is used as an advanced material which can be applied to various fields, such as an additive to chemotherapy, a regulator for a cell membrane, an agent for separating cells, an agent for tracking paths for labeled cells and other biological molecules, an agent for drug delivery, and a biosensor. However, there has been no report regarding an attempt to induce cell eradication by destroying a cell with a rapid spining of a nanowire imparted by an impression of a magnetic field.

Among the nanomedical technologies, the materials for which the most active research is conducted presently are noble metals and magnets. In case of noble metals, such as gold (Au) which has biocompatibility, they have an advantage that they can be utilized in a biotechnology field when nanotechnology is combined to them. Also, it is easy to impart, through a chemical or biological treatment, on a surface of gold, biofunctionality where biomaterials (nucleic acid and protein) can be attached to. In other words, a surface of gold is modified with a ligand, and disease marker factors, including a biomarker and linker, can be attached thereto. Accordingly, improvements can be made, using gold in a field of medical science, to high sensitivity diagnosis, assay, drug/gene delivery, and thermal treatment, and thus the research therefor is active. On the other hand, materials such as iron (Fe) are attractive as they have magnetic properties. Especially, studies have been widely performed to control a movement of a nanostructure using the magnetic properties in various fields of new applications including contrast medium of MRI, additives to a hyperthermia, a chemotherapy, and a radiotherapy for malignant cells, a cell membrane control, a magnetic separation, a cell arrangement, a tracking of paths for a labeled cell and other biological material, a drug delivery, a drug treatment, a genetic treatment and a nuclear radiation treatment for targeting a specific site, a nanoprobe, and a biosensor that are regarded as potential life science applications and potential medical science applications.

Studies concerning a barcode nanowire having a multilayer structure have been performed by various groups. The research group where the present inventors belong to has also continuously performed studies related to nanowires having biocompatibility, and filed Korean patent application No. 2006-0107410 (Korean granted patent No. 10-0848689; titled 'Multilayer Nanowire and Method for Producing the Same') as well as the US, Japanese, and European patent applications, and further filed Korean patent application No. 2008-0053146 (titled 'Core-shell Nanowire and Method for Producing the Same').

[Disclosure]

[Technical Problem]

The present invention is directed to providing a composition for inducing cell eradication, including a nanowire which can eradicate a cell with a physical spining force and a heat generated from a magnetically induced current of the nanowire under an AC magnetic field, and a method for cell eradication using the same.

[Technical Solution]

One aspect of the present invention provides a composition for inducing cell eradication, comprising a magnetic nanowire having a dipole, and a method for inducing cell eradication using the same.

[Advantageous Effects]

According to the present invention, when the magnetic nanowire having a dipole is used, it is possible to apply a physical impact on a cell with a spining of the nanowire introduced in a cell upon impressing a magnetic field, and additionally, a thermal treatment effect can be obtained with a heat generated by a magnetically induced current of the nanowire, therefore destroying the cell effectively.

[Description of Drawings]

FIG. 1 illustrates a schematic diagram of a cell eradication induction apparatus having four electrodes that emit the same AC frequency at a 90° phase shift from each other to cause cells containing internalized nanowires to spin.
FIG. 2(a) illustrates an optical microscope image for a magnetic nanowire suspended in a solution in which a dipole is formed. FIG. 2(b) illustrates a drawing showing a spinning of the nanowire by an externally-applied AC magnetic field.
FIG. 3 illustrates an optical microscope image showing rotation of cancer cells containing internalized nanowires caused by spinning of the magnetic nanowire therein by an externally-applied AC magnetic field.
FIG. 4 illustrates a result of quantified values of an inflammation response in accordance with an impression of an AC magnetic field in a magnetic nanowire internalized in a cell and an induction of a rotation.
FIG. 5 illustrates a schematic diagram showing a method for introducing a magnetic nanowire having a dipole in a tumor site in a mouse (FIG. 5A), and inducing necrosis of a tumor by impressing an AC magnetic field using a cell eradication induction apparatus having phase-shifted four electrodes.

[Modes of the Invention]

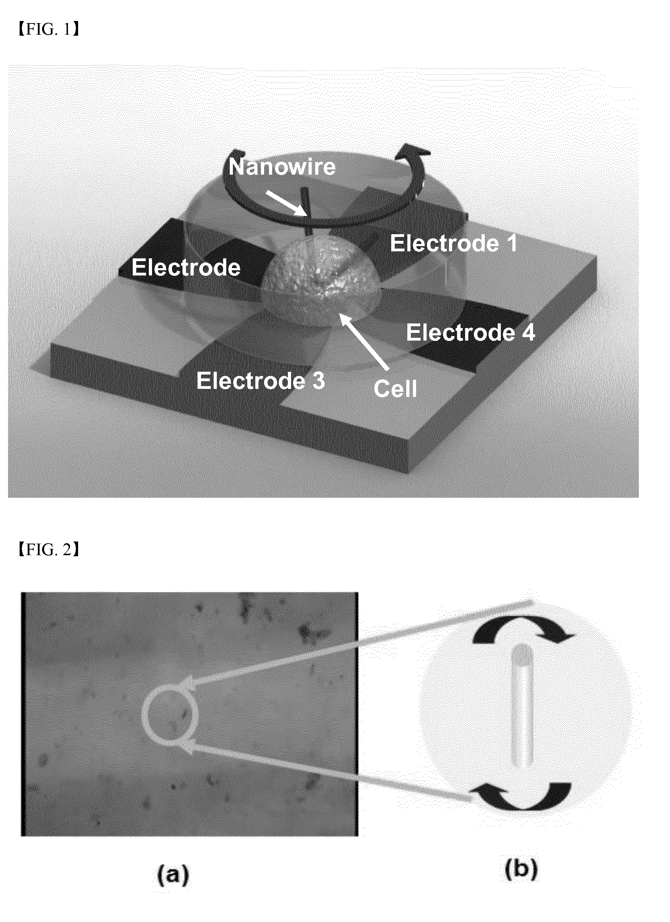

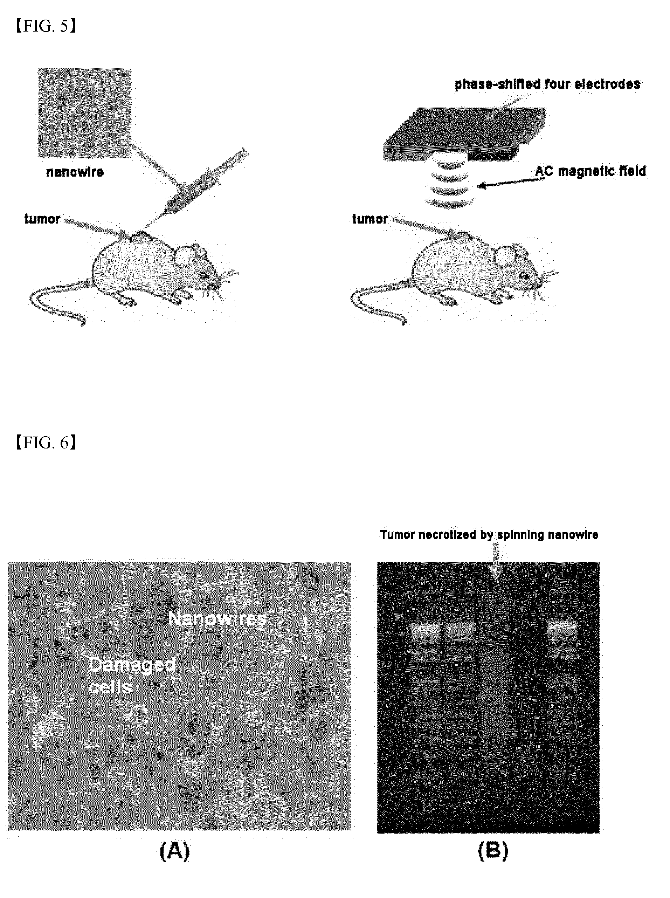

The present invention provides a composition for inducing cell eradication, comprising a magnetic nanowire having a dipole, and a method for inducing cell eradication using the magnetic nanowire having a dipole, namely a use of a magnetic nanowire having a dipole for inducing cell eradication.
A basic principle of the composition and method for inducing cell eradication using a magnetic nanowire having a dipole resides on destroying the cell membrane of a cell with a physical rotation force produced by spinning the nanowire under a magnetic field. Further, a magnetically induced current generated from the nanowire upon an impression of the magnetic field can perform an additional role in destroying the cell by the heat generated therefrom.
To explain a method for inducing cell eradication using a magnetic nanowire having a dipole, FIG. 1 can be referred as an example. FIG. 1 illustrates a schematic diagram of a cell eradication induction apparatus having phase-shifted four electrodes designed to rotate a nanowire by 90 degrees upon impressing an external AC magnetic field. Although FIG. 1 exemplarily shows that a cell in which a nanowire is introduced is positioned in the midst of an electrode, in practice, a magnetic nanowire is introduced into a tissue or cell which requires an induction of cell eradication in a living animal and a cell eradication apparatus having phase-shifted four electrodes is used as a means for spinning the magnetic nanowire, as exemplified in FIG. 5. The cell eradication apparatus having phase-shifted four electrodes used in FIG. 1 can be formed, for instance, by micro-patterning an Au thin layer on a substrate.
Examples of the nanowire used in the present invention include metal nanowires, magnet nanowires, or metal-magnet composite nanowires. In other words, provided the magnetic nanowire is mainly used, various nanowire structures, including a single magnetic nanowire, nanowire in which magnetic metal and non-magnetic metal materials are alloyed, nanowire having a core-shell form in which a non-magnet is coated on a surface of a magnet nanowire, and barcode nanowire in which a magnet and a non-magnet are alternatively laminated, can be designed to obtain desired composition and physical properties. Examples of the metals used in such a nanowire include not only noble metals, such as Au, Pt, and Pd, but also the metals, such as Fe, Cu, and A1 as well as their alloys. Meanwhile, examples of the magnet used in the nanowire include Fe, Co, Ni, Gd, their alloys, and their oxides. For instance, ferromagnetic materials, such as Fe, Co, Ni, Gd, and iron oxides can be used.
The nanowire can have a diameter of from 1 to 1000 nm, for instance, from 3 to 300 nm. Also, the nanowire can have a length of from 1 to 100 $\mu$m.
Meanwhile, dipoles must be formed in both ends of the nanowire so that the nanowire can rotate under a magnetic field. A dipole may denote positive and negative polars or electrodes facing each other. Therefore, the nanowire having a dipole according to the present invention may denote the one in which electric or magnetic dipoles are formed. In the present invention, an artificial formation of the dipole may be required depending on a material of the nanowire used. Namely, when a magnet is contained in a nanowire, dipoles may already be formed in both ends of the nanowire, and thus there is no need to artificially form the dipoles. However, in a case in which the dipoles are not formed in the nanowire itself, they can be formed through an electric or magnetic method. As an example, a magnetic nanowire can be formed in both ends of a non-magnetic metal nanowire, or a non-magnetic nanowire can be coated with a magnetic material. Methods of forming electric or magnetic dipoles are not particularly limited. For instance, electric dipoles can be formed in a metal nanowire using phase-shifted four electrodes under an AC electric field. Additionally, magnetic dipoles can be formed by means of magnetizing a nanowire including a ferromagnet into a permanent magnet. Accordingly, existence of the step of forming dipoles and method for such formation can vary depending on the material for a nanowire.

FIG. 2(a) illustrates an optical microscope image of a magnetic nanowire suspended in a solution in which a dipole is formed. FIG. 2(b) illustrates a drawing showing a rotation of the nanowire by an externally-applied AC magnetic field.

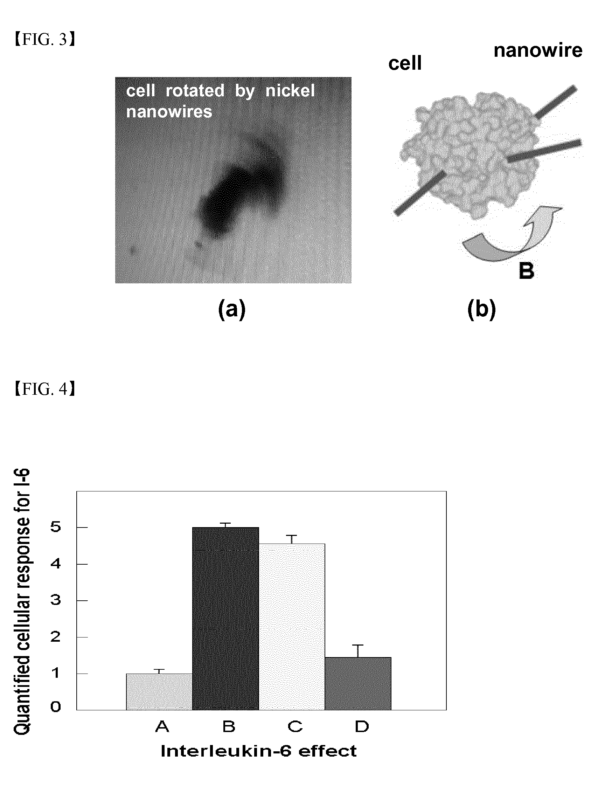

FIG. 3 illustrates an optical microscope image showing rotation of cancer cells containing internalized magnetic-nanowires caused by spinning of the magnetic nanowire therein by an externally-applied AC magnetic field. As confirmed by FIG. 3, a physical impact can be applied in a cell using a magnetic nanowire having a dipole by introducing the nanowire into the cell and spinning it. Further, the cell can be destroyed by a heat generated in the nanowire by a magnetically induced current.

An inflammation response is induced when an external impact is applied to a cell. Interleukin-6 is a kind of cytokines which induces an inflammation in relation to a trauma immune response and which may be used for quantifying a cell response depending on the produced amount. FIG. 4 illustrates an amount of the reaction of Interleukin-6 in accordance with the degree of an external stimulus, i.e. a rotation speed of a nanowire. The cell response showed its maximum value at 100 to 500 rpm of the nanowire's rotation speed. In this embodiment, the Interleukin-6 cell response of a human embryonic kidney cell (HEK-293) was observed. Interleukin-6 is a cytokine which induces an inflammation in relation to a trauma immune response. (A) is a cell response when a nanowire does not rotate, (B) is a cell response by rotating the nanowire with 100 rpm for 5 minutes, (C) is a cell response by rotating the nanowire with 500 rpm for about 5 minutes, (D) is a cell response by rotating the nanowire with 700 rpm for about 5 minutes, in which the cell responses are quantified by Interleukin-6, confirming that the cell response varies in accordance with the number of rotations per minute in the nanowire, and the cell response showed its maximum value at 100 to 500 rpm of the nanowire's rotation speed. It was addressed that Interleukin-6 cell response is maximized within the scope of the above number of rotations of the nanowire and destruction of a cell can occur effectively.

According to one embodiment of the present invention, the nanowire can be a barcode type nanowire having a multilayer structure. For example, the nanowire can be a barcode type magnet/noble metal multilayer nanowire. Noble metals, such as gold, can provide excellent biocompatibility, and biofunctionality to which a target-specific ligand such as an antibody can be attached, may be imparted therein. Further, a magnet enables the application in a magnetic resonance image. Also, both a magnet and noble metal enable a thermal treatment of cancer through the heat produced by an impression of a magnetic field. The barcode type nanowire having a multilayer structure and method for manufacturing the same are well known in the art. A Korean granted patent No. 10-0848689, which was previously filed by the present inventors and is incorporated herein by reference, discloses the details thereof.

According to one embodiment of the present invention, the nanowire can be a nanowire having a core-shell structure. For instance, the nanowire may consist of a magnet as a core nanowire and a noble metal as a shell layer. By incorporating a noble metal in a shell layer, biocompatibility and biofunctionality can be maximized. The nanowire having a core-shell structure and method for manufacturing the same are well known in the art. A Korean patent application No. 10-2008-0053146, which was previously filed by the present inventors and is incorporated herein by reference, discloses the details thereof.

The nanowire can be coated with a biocompatible polymer material, if needed. Also, the nanowire can be combined with a target-specific ligand. The target-specific ligand may denote a ligand which targets a tissue or cell to be treated by the nanowire according to the present invention so that the nanowire can be specifically introduced in the tissue or the cell. Examples of the target-specific ligand include not only nucleic acids, aptamers, antigens, and antibodies, but also ligand compounds which are known to be useful as they can be combined with particular receptors in a surface of a cell. Types of such nucleic acids, aptamers, antigens, antibodies, and ligand compounds are well known in the art. A bonding between the target-specific ligand and the nanowire or a coating layer coated on the nanowire can be attained by a physical bonding or a covalent bond between functional groups existing or formed on the nanowire or the coating layer. The functional groups may intrinsically exist on the nanowire, coating layer, or ligand, and further, the coating layer or ligand can be modified so as to have the functional groups which can be connected to each other, if necessary. The functional group existing on the coating layer and the functional group existing on the ligand can be selected from the examples of known combinations of functional groups so that they can form a bonding.

Meanwhile, a drug can be loaded on the nanowire. The nanowire to be rotated upon an impression of a magnetic field can move relatively freely than usual nanowires within blood vessels despite the flow of blood, and thus can be used as an effective drug delivering body. The form in which a drug is loaded on the nanowire is not particularly limited. For instance, when an antitumor agent is loaded on a nanowire, the nanowire according to the present invention can destroy cancer cells through not only a heat treatment and mechanical impact, but also chemotherapy.

Many methods regarding destroying cancer cells using nanomaterials have been proposed, yet the detailed methods of removing the nanomaterials from the human body after the destruction of cancer cells have not been suggested, and thus, there could be certain potential harm to sound cells, in which the harm may be caused by the remaining nanomaterials which were not discharged, although partial amount of the nanomaterials can be discharged out of the body with excreta. Use of the magnetic nanowire according to the present invention enables easy collection of the magnetic nanowire using a magnetic field, thereby solving above-described issues.

The composition for inducing cell eradication according to the present invention may comprise a carrier and a vehicle which are conventionally used in a domain of pharmaceuticals. In particular, examples thereof include ion exchange resin, alumina, aluminum stearate, lecithin, blood serum protein (e.g. human serum albumin), buffer material (e.g. phosphates, glycine, sorbic acid, potassium sorbate, partial glyceride mixture of saturated plant fatty acids), water, salts, or electrolytes (e.g. protamine sulfate, disodium hydrogen phosphate, potassium hydrogen phosphate, sodium chloride, and salts of zinc), colloidal silica, magnesium trisilicate, polyvinylpyrrolidone, cellulose-based substrate, polyethylene glycol, sodium carboxymethylcellulose, polyarylate, wax, polyethylene glycol, and wool grease, but the present invention is not limited thereto. The composition for inducing cell eradication according to the present invention may further include a lubricant, wetting agent, emulsifier, suspending agent, or preserving agent in addition to the above components.

According to one embodiment of the present invention, the composition for inducing cell eradication according to the present invention can be manufactured as an aqueous solution for parenteral administration, and preferably, a buffer solution, such as Hank's solution, Ringer's solution, or physically buffered saline water, can be used. Into an aqueous injection suspension, a substrate which can increase viscosity of the suspension, such as sodium carboxymethylcellulose, sorbitol, or dextran, can be added.

In another preferred embodiment of the present invention, the composition for inducing cell eradication can be a form of formulation for sterile injection, such as an aqueous or oil suspension for sterile injection. The suspension can be formulated according to the known technologies in the art using suitable a dispersant or wetting agent (e.g. Tween 80) and suspending agent. The formulation for sterile injection can also be a solution or suspension (e.g. solution in 1,3-butanediol) for sterile injection in a non-toxic diluent or solvent allowed for parenteral use. Examples of vehicles and solvents that can be used include mannitol, water, Ringer's solution, and isotonic sodium chloride solution. Further, sterile non-volatile oil is conventionally used as a solvent or suspending medium. Any type of non-volatile and non-irritant oil, including synthetic mono- and di-glyceride, can be used for the purpose hereby provided.

A step of administering the composition for inducing cell eradication to a living body can be performed through any path which is conventionally used in a domain of pharmaceuticals, preferably a parenteral administration, such as an administration through an intravenous, intra-abdominal, intramuscular, subcutaneous, or topical path.

Another aspect of the present invention relates to methods for inducing cell eradication using a magnetic nanowire having a dipole or a composition comprising the same. This aspect of the present invention provides methods for inducing cell eradication including administering a composition including a nanowire having a dipole to a subject, and impressing a magnetic field in the subject. In the present invention, the subject may denote a mammal including a human being. The magnetic nanowire having a dipole or a composition comprising the same according to the present invention can be particularly administered to a cell or tissue in which an induction of cell eradication is required. FIG. 4 illustrates a schematic diagram of a process of cell destruction as a result of thermal and mechanical impact when the composition comprising the magnetic nanowire is administered to the subject and an AC magnetic field is impressed therein. Both mechanical and thermal shocks can be used in the destruction of cell, and thus, efficiency of cell eradication induction can be increased.

According to one embodiment of the present invention, the method for inducing cell eradication can be performed using a cell eradication induction apparatus having phase-shifted four electrodes. Meanwhile, a rotation speed of the nanowire is preferably 100 to 500 rpm when the composition for inducing cell eradication according to the present invention is used in the treatment. To this end, the magnetic field may have a frequency of about 100 Hz to 1 MHz, but the present invention is not limited thereto. The rotation speed of the nanowire and the strength of the magnetic field can be properly adjusted by those of ordinary skill in the art.

The above and other objects, features, and advantages of the present invention will become more apparent to those of ordinary skill in the art with reference to Examples to be provided hereinafter. However, the present invention is not limited to Examples disclosed below, but may be implemented in various forms. The following Examples are described in order to enable those of ordinary skill in the art to embody and practice the present invention.

## EXAMPLES

### Preparatory Example: Preparation of nanowire

A nickel (Ni) nanowire was manufactured in the same manner with previous studies by the method to be described below. The nickel nanowire was manufactured by electro-depositing a nickel nanowire in nanopores of an anodized aluminum oxide (AAO) template having an average nominal pore diameter of 150 nm. The anodized aluminum oxide template used in the present invention was produced by anodizing an aluminum foil in 0.3 M oxalic acid. The anodized aluminum oxide had a plenty of pores. The pore had a diameter of 150 nm, and the pores were aligned uniformly. The electrolyte for electro-plating was a nickel electrobath comprising 1 g/L of nickel(II) chloride, 25g/L of Ni $(H_2NSO_3)_2$, and an $H_3BO_3$ solution. Before the electro-deposition of nickel, a conductive gold (Au) seed layer having a thickness of 200 nm was formed using thermal evaporation in a lower part of the anodized aluminum nanotemplate. This gold layer functioned as a cathode upon the electroplating. The conductive gold seed layer of the anodized aluminum nanotemplate was fixed on a glass slide using a carbon paste. Nickel ions were electroplated in the pores of the nanotemplate by impressing a current density of 35 mA/cm$^2$ at room temperature on the Au deposited in the lower part of the anodized aluminum nanotemplate, thereby forming a nanowire. A platinum (Pt)-niobium (Nb) electrode was used as a counter electrode. After depositing a nanowire, the alumina template was removed with 20 wt% of sodium hydroxide (NaOH) to separate each nickel nanowire. Then, the nickel nanowires were  sterilized with isopropyl alcohol (IPA), and were moved to Dulbecco's modified eagle medium (DMEM) culture medium in which 10 % fetal bovine serum (FBS) was added.

### Exemplary Principle: Principle of rotation of magnetic nanowire having a dipole

In a single nickel nanowire, the magnetization reversal is reversible to a given value (switching field, $H_{sw}$). At this point, the magnetization shifts to the opposite direction. This shift corresponds to the unstable states of magnetization. In case of nickel nanowires of a small diameter, the magnetization states can be described quantitatively as a function of the amplitude and the direction of the applied field in terms of the curling mode of magnetization reversal. Soft magnetic nanowires can switch in two different reversal modes, known as the transverse wall mode and the vortex wall mode, depending on their diameter. For thin nanowires, the reversal process occurs in the transverse wall mode. As for nanowires with large diameters, reversal take place in the vortex wall mode. In both modes the NWs switch by means of nucleation at the end of the NWs and subsequent propagation. For soft magnetic NWs, if the magnetocrystalline anisotropic energy is neglected, the domain wall energy is mainly composed of the demagnetization energy and exchange energy. The competition between these two energies determines the domain wall structure.

The domain wall energy can be written in polar coordinate as follows:

$$E = \int \left\{ A\left[ (\nabla \theta)^2 + (\nabla \vartheta)^2 \sin^2 \theta \right] + \varepsilon_m \right\} d_v \qquad (1)$$

where $\theta$ and $\vartheta$ are the polar angle and the azimuthal angle of magnetization, respectively. A is the exchange interaction energy density and $\varepsilon_m$ is the demagnetization energy density. The first term is the exchange energy. The second term denotes the demagnetization energy.

The transition from the transverse wall mode to the vortex wall mode occurs at the critical diameter where the energies of the transverse wall and vortex wall are equal. This force should rotate the magnetic nanowires to align their magnetic moments parallel to the local field and move toward higher magnetic field regions to minimize the magnetic energy of the nanowires. Therefore, the application of an external magnetic field can alter the position of cells with internalized ferromagnetic Ni nanowires.

### Example 1: Induction of cell eradication using a spinning nanowire

Suggested is a method for internalizing a nanowire in a cell and spinning the nanowire by controlling a magnetic field from outside in order to remove a living cell using a dynamic nanowire. In a system to rotate the nanowire according to one embodiment of the present invention, the cell containing the internalized nanowire is rotated using a cell eradication induction apparatus having phase-shifted four electrodes which emits the same AC frequency at each 90 degree phase-shift, as illustrated in FIG. 1. The microelectrodes were patterned by microfabrication of 150nm-thick gold film on glass substrates. In FIG. 1, it is explained as if the cell in which the nanowire is introduced is located at a center of the electrode for the purpose of explanation, yet in practice, the magnetic nanowire is introduced to a tissue or cell in a living animal where an induction of cell eradication is required, and a cell eradication apparatus having phase-shifted four electrodes is used as a means for spinning the magnetic nanowire, as illustrated in FIG. 5.

FIG.2 illustrates a nanowire's rotation effects generated by an externally-applied AC magnetic field. FIG. 2(a) is an optical microscope image of a magnetic nanowire suspended in a solution in which dipoles are formed. FIG. 2(b) is a schematic diagram showing a rotation of the nanowire upon an impression of a magnetic field. The resistance from the rotation of the cells was determined by the moment of inertia. When the cell was assumed to have a spherical shape (20 $\mu$m in diameter), the moment of inertia was $4\times10^{-25}$ kg m$^2$. The calculated torque ($\tau$) from spinning NWs ($\tau$ = **r** $\times$ **F**=$3.09\times10^{-23}$ Nm where r is the diameter of single cell and F is the force for rotation) could potentially move the cell. FIG. 3 is an optical microscope image showing a rotation of a nickel nanowire internalized in a human embryonic kidney cell (HEK-293) caused by an AC magnetic field. The spinning speed is approximately 500 rpm.

**Example 2: Destruction of a cell in accordance with a rotation speed of a nanowire controlled by an AC magnetic field.**

In order to observe a cell response of a human embryonic kidney cell (HEK-293) in accordance with a rotation speed of a nanowire controlled by an AC magnetic field, Interleukin-6 (I-6) cell response was observed with inducing a stimulus of a cell using the method of physically spinning the nanowire as suggested in the present invention. Interleukin-6 is a cytokine which induces an inflammation in relation to a trauma immune response. Interleukin-6 topically reacts to a specific tissue trauma and is a pro-inflammatory cytokine which is highly controlling particularly in a fibroblast cell. In order to reveal an inflammatory response of a cell against the nanowire, a gene-expression of Interleukin-6 is measured. As illustrated in FIG. 4, (A) is a cell response when a nanowire does not rotate, (B) is a cell response by rotating the nanowire with 100 rpm for 5 minutes, (C) is a cell response by rotating the nanowire with 500 rpm for about 5 minutes, (D) is a cell response by rotating the nanowire with 700 rpm for about 5 minutes, in which the cell responses are quantified by Interleukin-6, confirming that the cell response varies in accordance with the number of rotations per minute in the nanowire, and the cell response showed its maximum value at a range of 100 to 500 rpm of the nanowire's rotation speed. It was addressed that Interleukin-6 cell response is maximized within the scope of the above number of rotations of the nanowire, and that destruction of a cell can occur effectively. In a case in which only the nanowire in which a magnetic field is not applied is introduced, a substantial inflammation was not generated, but a cell survival rate in the fibroblast cell culture was observed to be decreasing up to 60 to 70% as the rotation speed increases from 100 rpm to 700 rpm upon an application of a magnetic field and as a rotation time increases.

An amplitude and rate of AC modulation are critical elements in determining a rotation of a nanowire in a cell, and the number of rotations of the nanowire can be changed by controlling an impulsed AC modulation. A short and strong stimulus is required for an effective treatment. Further, necrosis of a cancer cell can be induced by an increased topical temperature caused by the heat produced by a current induced by an impression of an AC magnetic field in a nickel nanowire which is a magnet, showing an advantageous combination of two methods for inducing necrosis of a cancer cell using one nanowire.

**Example 3: Induction of cancer cell eradication in a mouse having a pancreatic tumor using a spinning nanowire**

A cancer tumor was induced by injecting a pancreatic cancer cell in a healthy mouse. FIG. 5 illustrates a schematic diagram showing a method of introducing a magnetic nanowire having a dipole in a tumor site in a mouse and inducing necrosis of a tumor by impressing an AC magnetic field using a cell eradication induction apparatus having phase-shifted four electrodes. As explained in the above, the nanowire in an adequate concentration was injected, then the cell eradication induction apparatus having phase-shifted four electrodes was closely approached to the tumor containing the nanowire, and a rotation of the nanowire in the tumor was induced using the AC magnetic field. As a result, as illustrated in FIG. 6A, partial necrosis of cancer cells which were stained with hematoxylin and eosin was observed, and as shown in the DNA breakdown pattern gel image in FIG. 6B, a smeared pattern rather than laddering was observed, addressing that the destruction of cancer cells induced by spinning the nanowire occurred with a cell death mechanism by necrosis rather than cell apoptosis.

**Claims**

1. A composition for inducing cell eradication comprising a magnetic nanowire having a dipole.

2. The composition according to claim 1, wherein the nanowire is a metal nanowire, a magnet nanowire, or a metal/magnet composite nanowire.

**3.** The composition according to claim 2, wherein the metal is gold, platinum, palladium, copper, aluminum, or alloys thereof.

**4.** The composition according to claim 2, wherein the magnet is nickel, cobalt, iron, gadolinium, alloys thereof, or oxides thereof.

**5.** The composition according to claim 1, wherein the nanowire has a diameter of from 1 to 1000 nm and a length of from 1 to 100 $\mu$m.

**6.** The composition according to claim 1, wherein electric or magnetic dipoles are formed in both ends of the nanowire.

**7.** The composition according to claim 1, wherein the nanowire is a barcode type nanowire having a multilayer structure.

**8.** The composition according to claim 1, wherein the nanowire is a nanowire having a core-shell structure.

**9.** The composition according to claim 1, wherein the nanowire is bonded with a target-specific ligand.

**10.** The composition according to claim 1, wherein a drug is loaded on the nanowire.

**11.** The composition according to claim 1, wherein the nanowire is rotated upon an impression of an AC magnetic field.

**12.** A method for inducing cell eradication, comprising:

administering a composition comprising a nanowire having a dipole to a subj ect, and
impressing a magnetic field in the subject.

**13.** The method according to claim 12, wherein the impressing of a magnetic field in the subject is conducted using a cell eradication induction apparatus having a phase-shifted four electrodes.

【FIG. 1】

【FIG. 2】

(a)

(b)

【FIG. 3】

(a)

(b)

cell    nanowire

B

【FIG. 4】

【FIG. 5】

【FIG. 6】

(A)

(B)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20060107410
- KR 100848689
- KR 20080053146
- KR 1020080053146